# EUROPEAN PATENT APPLICATION

(11) **EP 4 282 381 A1**
(43) Date of publication of application: **29.11.2023**
(21) Application number: 21945691.0
(22) Date of filing: 20.08.2021
(51) Int. Cl.: A61F 2/24

(54) **IMPLANT DELIVERY APPARATUS AND IMPLANT DELIVERY SYSTEM**

(30) Priority: 17.06.2021 CN 202110672196
(71) Applicant: Shanghai Trulive Medtech Co., Ltd, Shanghai 201206 (CN); Jiangsu Trulive Medtech Co., Ltd, Rudong County Nantong Jiangsu 330038 (CN)
(72) Inventor: ZHANG, Wei, Shanghai 201206 (CN); LIU, Xiang, Shanghai 201206 (CN); ZHANG, Haijun, Shanghai 201206 (CN)
(74) Representative: Nordmeyer, Philipp Werner
(86) International application number: PCT/CN2021/113828
(87) International publication number: WO 2022/262110

(57) **Abstract**

An implant delivery device (10) and an implant delivery system are disclosed. The delivery device (10) includes a delivery assembly, a first flexible catheter (11) and a second flexible catheter (12). At least a part of the delivery assembly is movably inserted in the first flexible catheter (11) along an axial direction thereof, and both the first flexible catheter (11) and the delivery assembly are movably inserted in the second flexible catheter (12) along an axial direction thereof and configured to protrude out of the second flexible catheter (12) from its distal end. The first flexible catheter (11) and the second flexible catheter (12) are configured to bend independently. The delivery assembly changes its extension direction when bent shapes of the first flexible catheter (11) and the second flexible catheter (12) vary. Through section-wise bending of the flexible catheters, a large controllable bending angle can be achieved, solving the problems of difficult controllable bending and limited controllable bending angles associated with conventional delivery devices. As a result, implant release can be achieved in a narrow space in a safe and accurate manner, reducing the chance of paravalvular leakage and other issues that may arise during surgery.

## Description

### TECHNICAL FIELD

This invention relates to an implant delivery device and an implant delivery system.

### BACKGROUND

Heart valves are openable and closeable flap-like structures in the organs of humans and some animals. The human heart has four valves: the aortic valve connecting the left ventricle to the aorta; the pulmonary valve connecting the right ventricle to the pulmonary artery; the mitral valve connecting the left atrium to the left ventricle; and the tricuspid valve connecting the right atrium to the right ventricle. All these valves function like one-way valves which allow blood flow in the forward direction but prevent its backward flow.

With the development of social economy and the aging of population, the incidence of valvular heart disease has increased significantly. Studies have shown that this figure has reached up to 13.3% among those 75 years or older. At present, traditional surgical treatment remains first choice for patients with severe valvular disease. However, for those with advanced ages, complications in multiple organs, a history of thoracotomy or poor heart functions, the traditional surgical approach is associated with high risk and high mortality or even precludes some patients.

Transcatheter valve replacement usually makes use of a venous vascular access and often requires controllable bending of a delivery device at a large angle after it is inserted into the heart. For example, tricuspid valve replacement may involve inserting a delivery device through the inferior vena cava into the right atrium. This requires the device to be bendable at a large angle within a narrow space in a controlled manner. Conventional delivery devices can barely meet this requirement due to their limited controllable bending angles.

### SUMMARY OF THE INVENTION

It is an object of the present invention to overcome the problem of limited controllable bending angles associated with existing delivery devices by presenting an implant delivery device and an implant delivery system.

To this end, according to a first aspect of the present invention, there is provided an implant delivery device comprising a delivery assembly, a first flexible catheter and a second flexible catheter,
wherein at least a part of the delivery assembly is movably inserted in the first flexible catheter along an axial direction thereof, both the first flexible catheter and the delivery assembly movably inserted in the second flexible catheter along an axial direction thereof and configured to protrude out of the second flexible catheter from its distal end;
wherein the first and second flexible catheters are configured to bend independently, and wherein an extension direction of the delivery assembly changes when bent shapes of the first and second flexible catheters vary.

Optionally, in the implant delivery device, the delivery assembly may comprise a forward-backward driving unit and a loading unit,
wherein the loading unit is disposed at a distal end of the forward-backward driving unit,
wherein the forward-backward driving unit is movably inserted in the first flexible catheter along the axial direction thereof and is configured to protrude out of the first flexible catheter from its distal end.

Optionally, in the implant delivery device, the forward-backward driving unit may comprise a first delivery catheter and a second delivery catheter, and the loading unit may comprise a first loading tube and a second loading tube,
wherein the first delivery catheter is movably inserted in the second delivery catheter along an axial direction thereof, and wherein the second delivery catheter is movably inserted in the first flexible catheter along the axial direction thereof,
wherein at least a part of the first loading tube is movably inserted in the second loading tube along an axial direction thereof, and wherein: the first loading tube is coupled to a distal end of the first delivery catheter; the second loading tube is coupled to a distal end of the second delivery catheter; the first loading tube is configured for engagement with an implant; the second loading tube is configured to restrict a radial expansion of the implant; and the second loading tube has a radial outer dimension greater than a radial inner dimension of the first flexible catheter.

Optionally, in the implant delivery device, a radial outer dimension of the first loading tube may be greater than the radial inner dimension of the first flexible catheter and may be not greater than a radial outer dimension of the first flexible catheter.

Optionally, in the implant delivery device, the radial outer dimension of the second loading tube may be equal to the radial outer dimension of the first flexible catheter.

Optionally, in the implant delivery device, each of the first and second delivery catheters and the first and second flexible catheters may be a double-layer catheter, and wherein a braid wire is provided between the double-layer.

Optionally, in the implant delivery device, both the first and second loading tubes may be double-layer tubes.

Optionally, in the implant delivery device, the loading unit may further comprise a stent holder, wherein the stent holder is fixedly coupled to the loading unit and is used to engage with attachment lugs of the implant.

Optionally, the implant delivery device may further comprise a third delivery catheter, which is movably inserted in the delivery assembly along the axial direction of the first flexible catheter.

To the above end, according to a second aspect of the present invention, there is provided an implant delivery system comprising the implant delivery device as defined above and a driving device coupled to a proximal end of the delivery device. The driving device is configured to drive the first and second flexible catheters to bend respectively.

In summary, the present invention provides an implant delivery device and an implant delivery system. The implant delivery device includes a delivery assembly, a first flexible catheter and a second flexible catheter. At least a part of the delivery assembly is movably inserted in the first flexible catheter along an axial direction thereof, and both the first flexible catheter and the delivery assembly are movably inserted in the second flexible catheter along an axial direction thereof and are configured to protrude out of the second flexible catheter from its distal end. The first and second flexible catheters are configured to bend independently. The delivery assembly changes its direction of extension when bent shapes of the first and second flexible catheters vary.

With this arrangement, the first flexible catheter is movably inserted in the second flexible catheter, and the first and second flexible catheters may bend independently. Thus, through section-wise bending of these flexible catheters, a large controllable bending angle can be achieved, solving the problems of difficult controllable bending and limited controllable bending angles associated with conventional delivery devices. As a result, implant release can be achieved in a narrow space in a safe and accurate manner, reducing the chance of paravalvular leakage and other issues that may arise from inaccurate release during surgery.

### BRIEF DESCRIPTION OF THE DRAWINGS

Those of ordinary skill in the art would appreciate that the accompanying drawings are provided to facilitate a better understanding of the present invention and do not limit the scope thereof in any sense, in which:
Fig. 1 schematically illustrates an application scenario for implanting a prosthetic mitral valve stent;
Fig. 2 schematically illustrates an application scenario for implanting a prosthetic aortic valve stent;
Fig. 3 schematically illustrates an application scenario for implanting a prosthetic tricuspid valve stent;
Fig. 4 is a schematic illustration of a prosthetic tricuspid valve stent;
Fig. 5 is a schematic illustration of an implant delivery device according to an embodiment of the present invention;
Fig. 6 is a schematic enlarged partial view of the implant delivery device of Fig. 5;
Fig. 7a is a schematic illustration of a delivery assembly according to an embodiment of the present invention without a loading unit therein;
Fig. 7b is a schematic illustration of the delivery assembly according to another embodiment of the present invention with the loading unit therein;
Fig. 8 schematically illustrates a delivery assembly according to an embodiment of the present invention, which is being attached to an implant; and
Fig. 9 schematically illustrates release of an implant from an implant delivery device according to an embodiment of the present invention.

In these figures,
01, a prosthetic mitral valve stent; 02, a delivery device; 03, the inferior vena cava; 04, the right atrium; 05, the left atrium; 06, a prosthetic aortic valve stent; 07, the aorta; 08, a prosthetic tricuspid valve stent; 081, a stent; 082, a flange section; 083, a middle section; 084, a loading section; 085, attachment lugs;
10, a delivery device; 11, a first flexible catheter; 12, a second flexible catheter; 14, an forward-backward driving unit; 141, a first delivery catheter; 142, a second delivery catheter; 143, a third delivery catheter; 15, a loading unit; 151, a first loading tube; 152, a second loading tube; and 153, a stent holder.

### DETAILED DESCRIPTION

Objects, advantages and features of the present invention will become more apparent upon reading the following more detailed description of the present invention with reference to the accompanying drawings. Note that the figures are provided in a very simplified form not necessarily drawn to exact scale and for the only purpose of facilitating easy and clear description of the embodiments. In addition, structures shown in the figures are usually a part of actual structures. In particular, as the figures tend to have distinct emphases, they are often drawn to different scales.

As used herein, the singular forms "a", "an" and "the" include plural referents, unless the context clearly dictates otherwise. As used herein, the term "or" is generally employed in the sense of "and/or", "several" is generally employed in the sense of "at least one" and "at least two" is generally employed in the sense of "two or more", unless the context clearly dictates otherwise. Additionally, the use of the terms "first", "second" and "third" herein is intended for illustration only and is not to be construed as denoting or implying relative importance or as implicitly indicating the numerical number of the referenced item. Accordingly, defining an item with "first", "second" or "third" is an explicit or implicit indication of the presence of one or at least two of the items. As used herein, the term "proximal end" generally refers to an end closer to an operator, and the term "distal end" generally refers to an end closer to a lesion in a patient. The terms "one end" and "the other end", as well as "proximal end" and "distal end", are generally used to refer to opposite end portions including the opposite endpoints, rather than only to the endpoints. The terms "mounting", "coupling" and "connection" should be interpreted in a broad sense. For example, a connection may be a permanent, detachable or integral connection, a direct or indirect connection with one or more intervening media, or an internal communication or interaction between two units. As used herein, when an unit is referred to as being "disposed on" another unit, this is generally intended to only mean that there is a connection, coupling, engagement or transmission relationship between the two units, which may be either direct or indirect with one or more intervening units, and should not be interpreted as indicating or implying a particular spatial position relationship between the two units, i.e., the unit may be located inside, outside, above, under, beside, or at any other location with respect to the other unit, unless the context clearly dictates otherwise. Those of ordinary skill in the art can understand the specific meanings of the above-mentioned terms herein, depending on their context.

The present invention seeks to overcome the problem of limited controllable bending angles associated with existing delivery devices by presenting an implant delivery device and an implant delivery system.

This invention will be described below with reference to the accompanying drawings.

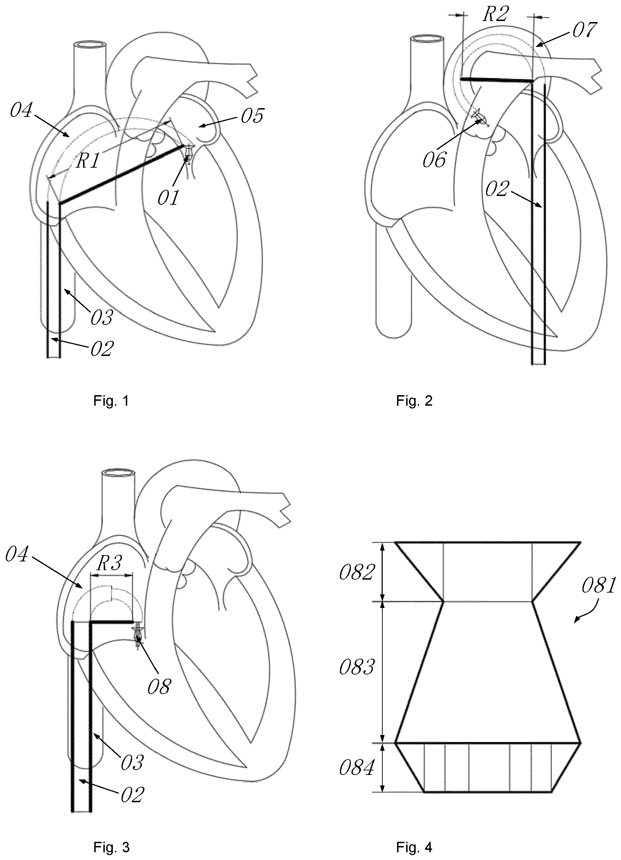

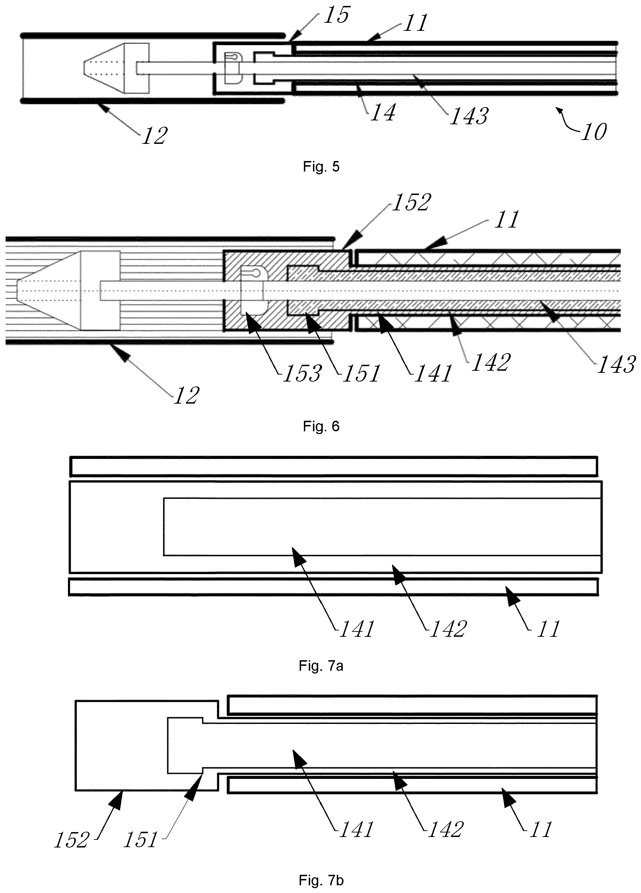

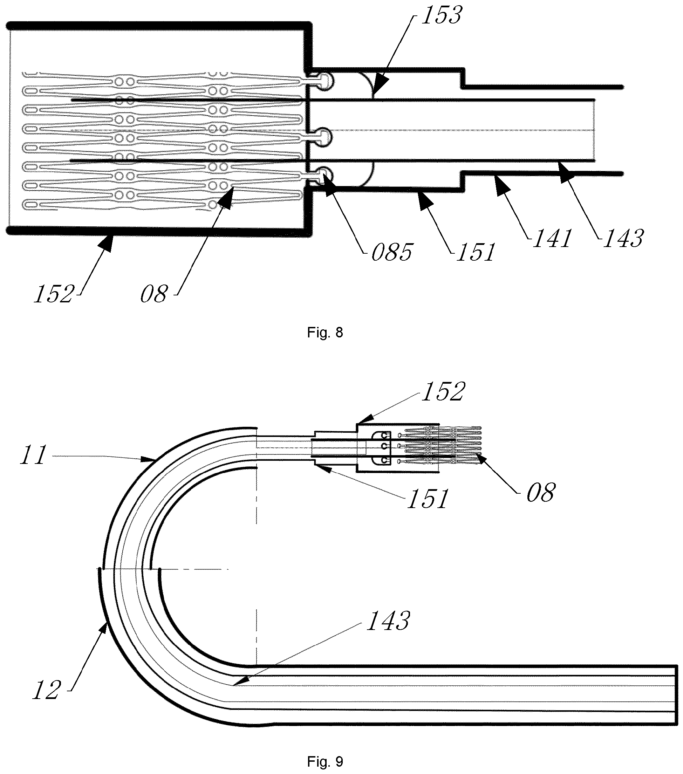

Reference is now made to Figs. 1 to 9. Fig. 1 schematically illustrates an application scenario for implanting a prosthetic mitral valve stent. Fig. 2 schematically illustrates an application scenario for implanting a prosthetic aortic valve stent. Fig. 3 schematically illustrates an application scenario for implanting a prosthetic tricuspid valve stent. Fig. 4 is a schematic illustration of a prosthetic valve stent. Fig. 5 is a schematic illustration of an implant delivery device according to an embodiment of the present invention. Fig. 6 is a schematic enlarged partial view of the implant delivery device of Fig. 5. Fig. 7a is a schematic illustration of a delivery assembly, in which a loading unit and a forward-backward driving unit have equal diameters. Fig. 7b is a schematic illustration of a delivery assembly according to an embodiment of the present invention, in which a loading unit and a forward-backward driving unit have different diameters. Fig. 8 schematically illustrates a delivery assembly according to an embodiment of the present invention, which is being attached to an implant. Fig. 9 schematically illustrates release of an implant from an implant delivery device according to an embodiment of the present invention.

Fig. 1 shows interventional implantation of a prosthetic mitral valve stent in an application scenario thereof. During the implantation of the prosthetic mitral valve stent 01, a delivery device 02 can be inserted into the right atrium 04 through the inferior vena cava 03 and then into the left atrium 05 through the interatrial septum. In this case, the delivery device 02 is allowed to have a relatively large bending diameter R1. Fig. 2 shows interventional implantation of a prosthetic aortic valve stent in an application scenario thereof. During the implantation of the prosthetic aortic valve stent 06, a delivery device 02 can be inserted through the aorta 07. In this case, the delivery device 02 is also allowed to have a relatively large bending diameter R2. However, as shown in Fig. 3, during the interventional implantation of a prosthetic tricuspid valve stent in an application scenario thereof, a delivery device 02 is required to be inserted into the right atrium 04 through the inferior vena cava 03 and bent and deflected within the right atrium 04 in order to release the prosthetic tricuspid valve stent 08 at the native tricuspid valve. In this case, the delivery device 02 is required to have a smaller bending diameter R3, which is smaller than the width of the right atrium 04.

On the basis of the above analysis, in embodiments of the present invention, there is provided an implant delivery device 10 suitable for use with various prosthetic valve stents. Referring to Figs. 5 and 6, in conjunction with Fig. 9, the delivery device 10 includes a delivery assembly, a first flexible catheter 11 and a second flexible catheter 12. At least a part of the delivery assembly is movably inserted in the first flexible catheter 11 along an axial direction thereof (which will curve as the first flexible catheter 11 bends). Both the first flexible catheter 11 and the delivery assembly are movably inserted in the second flexible catheter 12 along an axial direction thereof (which will likewise curve as the second flexible catheter 12 bends) and configured to protrude out of the second flexible catheter 12 from its distal end. The first flexible catheter 11 and the second flexible catheter 12 are configured to bend independently. The direction of extension of the delivery assembly will change when the first flexible catheter 11 and the second flexible catheter 12 bend. The second flexible catheter 12 forms an outermost layer of the delivery device 10, and the first flexible catheter 11 is located within the second flexible catheter 12. Both the first flexible catheter 11 and the second flexible catheter 12 are preferred to be double-layer catheters, and a braid wire is sandwiched between the two layers of each double-layer catheter. The braid wires may be pulled and controlled around proximal ends of the first flexible catheter 11 and the second flexible catheter 12 to make controllable adjustments to bent shapes of distal end portions of the first flexible catheter 11 and the second flexible catheter 12 (i.e., achieve controlling bending thereof). Those skilled in the art can configure the structures of the first flexible catheter 11 and the second flexible catheter 12 based on common general knowledge in the art. In particular, both the first flexible catheter 11 and the second flexible catheter 12 may be structured like the active catheter disclosed in Chinese Patent Publication No. CN212593458U, the entirety of which is hereby incorporated by reference.

Referring to Figs. 3 and 9, in an exemplary implant release embodiment, the distal end of the second flexible catheter 12 is inserted into the right atrium 04 through the inferior vena cava 03 and controllably bent therein, for example, through 90°. After that, the first flexible catheter 11 may be pushed out of the bent second flexible catheter 12 and controllably bent, for example, also through 90°. In this way, the entire delivery device 10 achieves an overall controllable bending angle of 180°. Further, the delivery assembly, which carries the implant therein and has changed its direction of extension as a result of the bending of the first flexible catheter 11 and the second flexible catheter 12, is advanced so that its distal end protrudes out of the distal end of the first flexible catheter 11 and moved to a suitable location, followed by release of the implant. For details of retrieval of the implant, reference can be made to the above description of the implant release process, and repeated description thereof is omitted. In this way, through movably inserting the first flexible catheter 11 in the second flexible catheter 12 and designing the first flexible catheter 11 and the second flexible catheter 12 as individually controllably bendable components, these flexible catheters entail a section-wise bending approach, which allows a large controllable bending angle, solving the problems of difficult controllable bending and limited controllable bending angles associated with conventional delivery devices. As a result, implant release can be achieved in a narrow space in a safe and accurate manner, reducing the chance of paravalvular leakage and other issues that may arise from inaccurate release during surgery. It would be understood that the implant delivery device 10 of this embodiment is not limited to being used for the implantation of an implant at the tricuspid valve. Instead, it can also be used to implant an implant at the aortic valve, the mitral valve or other sites.

The following description is set forth with reference to Fig. 4 in the context of the implant being implemented as a prosthetic tricuspid valve stent 08 as an example. The prosthetic tricuspid valve stent 08 includes a stent 081, a valve and a skirt. The stent 081 includes a flange section 082, a middle section 083 and a loading section 084. The flange section 082 is configured to be released at the valve annulus and essentially intended to provide support to the prosthetic tricuspid valve stent 08 to avoid it from falling off into a ventricle. Moreover, the skirt covers an inner surface and/or an outer surface of the flange section 082, enabling it to further provide a sealing effect. The middle section 083 has significant radial and axial stiffness, which enables it to withstand traction from leaflets of the valve. The middle section 083 is made up of structural units which are axially changeable in shape, such as mesh-like, wave-shaped or other structural units. At least one row of structural units is arranged along an axial direction of the middle section, and all structural units in each row are circumferentially joined together. In case of multiple rows, the units in different rows may be connected directly or indirectly in the axial direction. Mesh-like structural units are preferred, which may be triangular, rhombic, pentagonal, teardrop-shaped or otherwise-shaped closed mesh cells, with rhombic mesh cells being more preferred. The loading section 084 is configured to be connected with the delivery device 10, ensuring that the prosthetic tricuspid valve stent 08 is maintained stationary with respect to the delivery device 10 during loading of the valve into the delivery device 10, release and separation of the valve from the delivery device 10 and movement of the valve being loaded in the delivery device 10 within the patient's body. The loading section 084 is provided with connecting structures for connecting it to the delivery device 10, such as attachment lugs 085 (see Fig. 8) or fixation lugs. In this way, the prosthetic tricuspid valve stent 08 is secured to the delivery device 10 by the connecting structures, during loading and advancement of the valve. When the valve is delivered to a target site, the connecting structures are detached from the delivery device 10, allowing release of the prosthetic tricuspid valve stent 08.

Optionally, the stent 081 may be made of, for example, nitinol, a titanium alloy, a cobalt-chromium alloy, MP35n, 316 stainless steel, L605, Phynox/Elgiloy, a platinum-chromium alloy, or another biocompatible metal known to those skilled in the art. Optionally, the stent 081 may be alternatively made of an elastic or plastically deformable material, such as a balloon expandable material, or a shape memory alloy which can transition in response to temperature changes between a collapsed configuration for delivery and an expanded or unfolded configuration. Preferably, the stent 081 is fabricated by cutting a nickel-titanium alloy tube with an outer diameter of 5-15 mm. The stent 081 may have a final diameter chosen according to the actual demand.

The valve includes at least two prosthetic leaflets, which may be made of, for example, animal pericardium or another biocompatible polymeric material. Each prosthetic leaflet is directly or indirectly attached to the stent 081 firmly at one end and free at the other end. During operation of the valve, the prosthetic leaflets open and close the blood flow passageway in place of the native leaflets of the tricuspid valve.

The skirt covers the inner and/or outer surface(s) of the stent 081, providing a sealing effect which ensures one-way blood flow from an inflow end to outflow end of the stent. The skirt may be made of, for example, pericardium or another biocompatible polymeric material commonly used in the art, such as polyethylene terephthalate (PET), polytetrafluoroethylene (PTFE). However, the present invention is not limited to any particular material of the skirt. It is to be noted that the above-discussed prosthetic tricuspid valve stent 08 is merely a non-limiting example of the implant, and those skilled in the art can understand and modify its structure and operating mechanism based upon common general knowledge in the art. In some other embodiments, those skilled in the art may select other suitable implant, such as the prosthetic mitral valve disclosed in Chinese Patent Publication No. CN204709077U or the prosthetic aortic valve disclosed in Chinese Patent Publication No. CN104000672A. The entire contents of these applications are hereby incorporated by reference herein. It would be understood that the delivery device 10 of this embodiment is also applicable to other implants such as prosthetic mitral valves and prosthetic aortic valves, and the present invention is not limited in this regard.

The implant delivery device 10 of this embodiment is further described below with reference to Figs. 5 and 6. In the delivery device 10, the delivery assembly includes a forward-backward driving unit 14 and a loading unit 15. The loading unit 15 is disposed at a distal end of the forward-backward driving unit 14, and the forward-backward driving unit 14 is movably inserted in the first flexible catheter 11 along the axial direction thereof, and a distal end of forward-backward driving unit 14 is configured to protrude out of the distal end of the first flexible catheter 11. A proximal end of the forward-backward driving unit 14 may be coupled to, for example, a control component such as a handle. Under the control of the proximal control component, the forward-backward driving unit 14 is able to move forth and back in the axial direction of the first flexible catheter 11, causing the loading unit 15 disposed at its distal end to move in the same way. The loading unit 15 is configured to load the implant and is connected to, for example, the above-discussed loading section 084 of the prosthetic tricuspid valve stent 08.

Referring to Fig. 8, optionally, the loading unit 15 further includes a stent holder 153 fixedly coupled to the loading unit 15. The stent holder 153 is configured for attachment to the attachment lugs 085 of the implant. The stent holder 153 may be formed of, for example, a metallic material and may be fixed to the loading unit 15 by adhesive bonding. The stent holder 153 comprises sockets for mating with the attachment lugs 085, and the attachment lugs 085 can be inserted and retained within the sockets as a result of distal movement of the loading unit 15. With the implant being retained in place, the attachment lugs 085 can be removed from the sockets by proximally pulling the loading unit 15, thereby releasing the implant.

Referring to Fig. 7a, in one embodiment, the forward-backward driving unit 14 includes a first delivery catheter 141 and a second delivery catheter 142. The first delivery catheter 141 is movably inserted in the second delivery catheter 142 along an axial direction thereof, which is in turn movably inserted in the first flexible catheter 11 along the axial direction thereof. Moreover, the distal end of the forward-backward driving unit 14 is configured as the loading unit 15, dispensing with a separate loading unit 15. A distal end of the first delivery catheter 141 is configured to be coupled to the implant, and the second delivery catheter 142 is configured to restrict a radial expansion of the implant. The proximal ends of the first delivery catheter 141 and the second delivery catheter 142 may be coupled to a driving device such as a handle, and under the control of the driving device, the first delivery catheter 141 and the second delivery catheter 142are driven to move forth and back in the axial direction of the first flexible catheter 11.

The following description is set forth in the context of all the first delivery catheter 141, the second delivery catheter 142 and the first flexible catheter 11 being implemented as round tubes as an example. The second delivery catheter 142 has an inner diameter greater than an outer diameter of the first delivery catheter 141, and inner surface of the distal end of the first delivery catheter 141 is adhesively bonded to the stent holder 153. Once the stent holder 153 is attached to the implant, as a result of proximal movement of the first delivery catheter 141 (e.g., to the right in Fig. 7a) with respect to the second delivery catheter 142, the wall of the second delivery catheter 142 will restrict radial expansion of the implant. Thus, the implant can be compressed and loaded between the first delivery catheter 141 and the second delivery catheter 142. Generally, in the compressed configuration of the implant, since the flange section 082 and the middle section 083 each have a greater diameter than the loading section 084, the implant substantially has a shape which is flared distally (i.e., toward the left in Fig. 7a). To this end, it is desired to leave a clearance between an outer wall of the first delivery catheter 141 and an inner wall of the second delivery catheter 142 to facilitate loading of the implant. Since the delivery assembly of this embodiment is constructed simply from several tubes, it has a simple structure and can be easily manufactured.

Referring to Fig. 7b, in another embodiment, the forward-backward driving unit 14 includes a first delivery catheter 141 and a second delivery catheter 142, and the loading unit 15 includes a first loading tube 151 and a second loading tube 152. The first delivery catheter 141 is movably inserted in the second delivery catheter 142 along an axial direction thereof, and the second delivery catheter 142 is movably inserted in the first flexible catheter 11 along the axial direction thereof. At least a part of the first loading tube 151 is inserted in the second loading tube 152 along an axial direction thereof. The first loading tube 151 is connected to a distal end of the first delivery catheter 141, and the second loading tube 152 is connected to a distal end of the second delivery catheter 142. The first loading tube 151 is configured for attachment to the implant, and the second loading tube 152 is configured to restrict a radial expansion of the implant. A radial outer dimension of the second loading tube 152 is greater than a radial inner dimension of the first flexible catheter 11. It is to be noted that, here, the radial outer dimension refers to a maximum radial outer dimension of the tube. For example, if the tube is round, then the radial outer dimension refers to its outer diameter. If the tube is polygonal, then the radial outer dimension refers to an outer diameter of a circumscribed circle of the polygon. Moreover, the radial inner dimension refers to a minimum radial inner dimension of the tube. For example, if the tube is round, then the radial inner dimension refers to its inner diameter. If the tube is polygonal, then the radial inner dimension refers to an inner diameter of an inscribed circle of the polygon.

The following description is set forth with reference to Fig. 8 in the context of all the first delivery catheter 141, the second delivery catheter 142, the first loading tube 151 and the second loading tube 152 being implemented as round tubes as an example. In this embodiment, the first delivery catheter 141 and the second delivery catheter 142 are configured only to be driven by a proximal driving device to move forth and back in the axial direction of the first flexible catheter 11, but are not directly involved in the loading or restriction of the implant. Instead, the loading and the radial expansion restriction of the implant are accomplished by the first loading tube 151 and the second loading tube 152. Referring to Figs. 7b and 8, an inner wall of the distal end of the first loading tube 151 is adhesively bonded to the stent holder 153, and the first loading tube 151 is configured to be coupled to the loading section 084 of the implant (e.g., to the attachment lugs 085). The second loading tube 152 is configured to restrict radial expansion of the implant. When the first delivery catheter 141 moves proximally with respect to the second delivery catheter 142, the first loading tube 151 will also move proximally, together with the implant being carried thereon. As a result, expansion of the implant is restricted by the second loading tube 152, and the implant is compressed and loaded into the second loading tube 152. On the contrary, as a result of distal movement of the first delivery catheter 141 with respect to the second delivery catheter 142, the first loading tube 151 will also move distally, causing the implant carried thereon to move out of the second loading tube 152. Subsequently, the implant expands by itself (e.g., self-expanding stent) or is expanded (e.g., under the action of a balloon), depending on its design.

In the embodiment shown in Fig. 7a, a maximum outer diameter of the delivery assembly is the outer diameter of the second delivery catheter 142, and an inner diameter of the second delivery catheter 142 must be greater than a maximum outer diameter of the implant. Moreover, the inner diameter of the first flexible catheter 11 is greater than the outer diameter of the second delivery catheter 142, and an inner diameter of the second flexible catheter 12 is greater than an outer diameter of the first flexible catheter 11. Consequently, the delivery device 10 has a relatively large outer diameter, which makes it unsuitable for use with some accesses and thus limits its scope of application.

A comparison is drawn between the embodiments shown in Figs. 7a and 7b, in which it is assumed that the same implant is loaded and corresponding components in these embodiments have the same wall thicknesses. Accordingly, the second loading tube 152 of Fig. 7b has the same outer diameter as the second delivery catheter 142 of Fig. 7a. Moreover, since the inner diameter of the first flexible catheter 11 of Fig. 7b is smaller than the outer diameter of the second loading tube 152, the outer diameter of the first flexible catheter 11 of Fig. 7b is smaller than the outer diameter of the first flexible catheter 11 of Fig. 7a, and the inner diameter of the second flexible catheter 12 is greater than both the outer diameters of the first flexible catheter 11 and the second loading tube 152. Therefore, compared with the embodiment shown in Fig. 7a, the second flexible catheter 12 in the embodiment shown in Fig. 7b is allowed to have an effectively reduced inner diameter, which can result in a reduction in the maximum outer diameter of the delivery device 10 and impart improved passage performance and applicability to the delivery device 10.

Further, a radial outer dimension of the first loading tube 151 is greater than the radial inner dimension of the first flexible catheter 11 and is not greater than a radial outer dimension of the first flexible catheter 11. With the radial outer dimension of the first loading tube 151 being greater than the radial inner dimension of the first flexible catheter 11, a distal space of the first flexible catheter 11 can be fully used. Moreover, with the radial outer dimension of the first loading tube 151 being not greater than the radial outer dimension of the first flexible catheter 11, an excessively large outer diameter of the first loading tube 151, and hence an excessively large outer diameter of the second loading tube 152, can be avoided, dispensing with the need to increase the inner diameter of the second flexible catheter 12. Preferably, the second loading tube 152 has the same outer diameter as the first flexible catheter 11, while the outer diameter of the first loading tube 151 is slightly greater than the inner diameter of the first flexible catheter 11. With this arrangement, outer surfaces of the second loading tube 152 and the first flexible catheter 11 will be flush with each other, allowing the second flexible catheter 12 to have a relatively small inner diameter.

It would be appreciated that, while the first delivery catheter 141, the second delivery catheter 142, the first loading tube 151 and the second loading tube 152 have been described in the above embodiment as round tubes, the present invention is not so limited. In practice, those skilled in the art may replace one or more of them with tubes having other cross-sectional shapes, such as oblong tubes, without departing from the scope of the present invention. Optionally, both the first delivery catheter 141 and the second delivery catheter 142 are double-layer catheters, and a braid wire is sandwiched between the two layers. In this case, controllable bending of the first delivery catheter 141 and the second delivery catheter 142 can be achieved in an active manner to make adjustments to their distal bent shapes by manipulating the proximal driving device. Preferably, both the first loading tube 151 and the second loading tube 152 are double-layer tubes. The first loading tube 151 may be of the same structure and fabricated from the same material as the first delivery catheter 141 and differ therefrom only in having a different diameter. The second loading tube 152 may be of the same structure and fabricated from the same material as the second delivery catheter 142 and differ therefrom only in having a different diameter.

In an exemplary embodiment, inner layers of the first delivery catheter 141 and the first loading tube 151 may be made of a material selected from, for example, PE, PP, PI, PEEK, Pebax, PA, TPU or PC, and their outer layers may be made of a material selected from, for example, PE, PP, PI, PEEK, Pebax, PA11, PA12, TPU or PC. The first loading tube 151 is preferred to have an inner diameter lying between 7 mm and 9 mm, and the first delivery catheter 141 is preferred to have an inner diameter lying between 6 mm and 8 mm. The inner and outer layers of the second delivery catheter 142, the second loading tube 152, the first flexible catheter 11 and the second flexible catheter 12 may be made of at least one selected from PE, PP, PI, PEEK, Pebax, PA11, PA12, TPU and PC. It would be appreciated that the second delivery catheter 142, the second loading tube 152, the first flexible catheter 11 and the second flexible catheter 12 may be made of either the same or different materials. Moreover, the inner and outer layers of each of the second delivery catheter 142, the second loading tube 152, the first flexible catheter 11 and the second flexible catheter 12 may be made of either the same or different materials. The second loading tube 152 is preferred to have an inner diameter lying between 8 mm and 11 mm, and the inner diameter of the second delivery catheter 142 is preferred to lie between 6 mm and 9 mm. The inner diameter of the first flexible catheter 11 is preferred to lie between 8 mm and 11 mm, and the inner diameter of the second flexible catheter 12 is preferred to lie between 9 mm and 12 mm. The second flexible catheter 12 is preferred to have an outer diameter lying between 10 mm and 13 mm. Further, in each of the double-layer first delivery catheter 141, double-layer second delivery catheter 142, double-layer first loading tube 151, double-layer second loading tube 152, double-layer first flexible catheter 11 and double-layer second flexible catheter 12, the braid wire provided between the two layers may be chosen as a polymeric wire made of PA or PET, or as a metallic wire made of SS304 or SS316 stainless steel or the like. The braid wire is preferred to have a diameter lying between 0.1 mm and 1 mm. It would be appreciated that the materials and dimensions of the first delivery catheter 141, the second delivery catheter 142, the first loading tube 151, the second loading tube 152, the first flexible catheter 11 and the second flexible catheter 12 described in the above exemplary embodiment is merely for illustration, and the present invention is not limited to any particular materials or dimensions of these components. Those skilled in the art can modify the materials and dimensions of the tubes to adapt them to practical needs.

Preferably, the delivery device 10 further includes a third delivery catheter 143, which is movably inserted in the delivery assembly along the axial direction of the first flexible catheter 11. More particularly, the third delivery catheter 143 may be movably inserted in the first delivery catheter 141 along the axial direction of the first flexible catheter 11. The third delivery catheter 143 is configured for passage of a guidewire therethrough and for guidance of advancement and retraction of the guidewire during delivery. In an exemplary embodiment, the third delivery catheter 143 is a single-layer catheter made of a material selected from PE, PP, PI, PEEK, Pebax, PA11, PA12, TPU, PC and the like, and the present invention is not particularly limited in this regard. The third delivery catheter 143 has an inner diameter preferred to lie between 2 mm and 6 mm.

Steps in a process for implanting the prosthetic tricuspid valve stent 08 using the delivery device 10 of the present embodiment are described below as an example.

In step 1, referring to Fig. 8, the prosthetic tricuspid valve stent 08 is loaded into the delivery device 10. Specifically, the attachment lugs 085 of the prosthetic tricuspid valve stent 08 are engaged with the stent holder 153, and the second delivery catheter 142 is distally pushed with respect to the first delivery catheter 141, causing the second loading tube 152 to radially compress the prosthetic tricuspid valve stent 08 until the prosthetic tricuspid valve stent 08 is entirely received within the second loading tube 152.

In step 2, referring to Figs. 3 and 9, once the prosthetic tricuspid valve stent 08 is loaded, the delivery device 10 is inserted with the stent being retained therein through the inferior vena cava into the right atrium. After reaching a target site, the second flexible catheter 12 is first controllably bent (e.g., through 90°), and the first flexible catheter 11 is pushed out of the distal end of the second flexible catheter 12 together with the delivery assembly. The first flexible catheter 11 is then controllably bent (e.g., through 90°) so that the two flexible catheters achieve an overall controllable bending angle of 180°.

In step 3, the prosthetic tricuspid valve stent 08 is released. A distal portion of the delivery assembly is adjusted in position so that the prosthetic tricuspid valve stent 08 is aligned with the target site. After that, the first delivery catheter 141 is maintained axially stationary, and the second delivery catheter 142 is proximally retracted, releasing the flange section 082 of the prosthetic tricuspid valve stent 08 from the second loading tube 152. The flange section 082 then radially expands and anchors to the annulus of the native tricuspid valve. Afterwards, the second delivery catheter 142 is further retracted, releasing the middle section 083 of the prosthetic tricuspid valve stent 08 from the second loading tube 152. Subsequently, the first delivery catheter 141 is proximally retracted to detach the attachment lugs 085 from the stent holder 153, completing the release of the prosthetic tricuspid valve stent 08.

The prosthetic tricuspid valve stent 08 can be retrieved by steps similar to the above implantation and release steps. First of all, the delivery device 10 is inserted into the right atrium through the inferior vena cava. The first flexible catheter 11 and the second flexible catheter 12 are then successively controllably bent, and the distal portion of the delivery assembly is adjusted in position so that it is aligned with the prosthetic tricuspid valve stent 08. The first delivery catheter 141 is then distally pushed, engaging the stent holder 153 with the attachment lugs 085 of the prosthetic tricuspid valve stent 08. Subsequently, the prosthetic tricuspid valve stent 08 is loaded into the second loading tube 152 by distally pushing the second delivery catheter 142 and proximally retracting the first delivery catheter 141. The delivery device 10 is then withdrawn, completing the retrieval of the prosthetic tricuspid valve stent 08.

In this embodiment, there is also provided an implant delivery system, which includes the implant delivery device 10 as defined above and a driving device (not shown) coupled to a proximal end of the delivery device 10. The driving device is used to drive the first flexible catheter 11 and the second flexible catheter 12 to bend. The driving device may be, for example, a handle or another component. It may be coupled to both the braid wires in the first flexible catheter 11 and the second flexible catheter 12 so as to be able to drive and cause bending of the first flexible catheter 11 and the second flexible catheter 12. In addition, the driving device may be further used to control and drive bending of the first delivery catheter 141 and the second delivery catheter 142 so as to enable positional adjustments to the distal end of the delivery assembly. These skilled in the art can configure the structure of the driving device based on common general knowledge in the art, and further description thereof is omitted herein. Since the delivery system incorporates the above-discussed delivery device 10, it has the benefits that the delivery device 10 provides.

In summary, the present invention provides an implant delivery device and an implant delivery system. The implant delivery device includes a delivery assembly, a first flexible catheter and a second flexible catheter. At least a part of the delivery assembly is movably inserted in the first flexible catheter along an axial direction thereof, and both the first flexible catheter and the delivery assembly are movably inserted in the second flexible catheter along an axial direction thereof and configured to protrude out of the second flexible catheter from its distal end. The first and second flexible catheters are configured to bend independently. The delivery assembly changes its direction of extension when bent shapes of the first and second flexible catheters vary. With this arrangement, the first flexible catheter is movably inserted in the second flexible catheter, and the first and second flexible catheters are bendable independently. Thus, through section-wise bending of these flexible catheters, a large controllable bending angle can be achieved, solving the problems of difficult controllable bending and limited controllable bending angles associated with conventional delivery devices. As a result, implant release can be achieved in a narrow space in a safe and accurate manner, reducing the chance of paravalvular leakage and other issues that may arise from inaccurate release during surgery.

It is to be noted that the foregoing several embodiments are not limited to be used alone but can be combined. The present invention is not limited in this regard. The description presented above is merely that of some preferred embodiments of the present invention and does not limit the scope thereof in any sense. Any and all changes and modifications made by those of ordinary skill in the art based on the above teachings are intended to fall within the scope of the present invention.

## Claims

1. An implant delivery device, comprising a delivery assembly, a first flexible catheter and a second flexible catheter,
wherein at least a part of the delivery assembly is movably inserted in the first flexible catheter along an axial direction thereof, each of the first flexible catheter and the delivery assembly movably inserted in the second flexible catheter along an axial direction thereof and configured to protrude out from a distal end of the second flexible catheter;
wherein the first and second flexible catheters are configured to bend independently, and wherein an extension direction of the delivery assembly changes when bent shapes of the first and second flexible catheters vary.

2. The implant delivery device according to claim 1, wherein the delivery assembly comprises a forward-backward driving unit and a loading unit,
wherein the loading unit is disposed at a distal end of the forward-backward driving unit, and
wherein the forward-backward driving unit is movably inserted in the first flexible catheter along the axial direction thereof and is configured to protrude out from a distal end of the first flexible catheter.

3. The implant delivery device according to claim 2, wherein the forward-backward driving unit comprises a first delivery catheter and a second delivery catheter, and wherein the loading unit comprises a first loading tube and a second loading tube,
wherein the first delivery catheter is movably inserted in the second delivery catheter along an axial direction thereof, and wherein the second delivery catheter is movably inserted in the first flexible catheter along the axial direction thereof,
wherein at least a part of the first loading tube is movably inserted in the second loading tube along an axial direction thereof, and wherein: the first loading tube is coupled to a distal end of the first delivery catheter; the second loading tube is coupled to a distal end of the second delivery catheter; the first loading tube is configured for engagement with an implant; the second loading tube is configured to restrict a radial expansion of the implant; and the second loading tube has a radial outer dimension greater than a radial inner dimension of the first flexible catheter.

4. The implant delivery device according to claim 3, wherein a radial outer dimension of the first loading tube is greater than the radial inner dimension of the first flexible catheter, and is not greater than a radial outer dimension of the first flexible catheter.

5. The implant delivery device according to claim 3, wherein the radial outer dimension of the second loading tube is equal to the radial outer dimension of the first flexible catheter.

6. The implant delivery device according to claim 3, wherein each of the first and second delivery catheters and the first and second flexible catheters is a double-layer catheter, and wherein a braid wire is provided between the double-layer.

7. The implant delivery device according to claim 6, wherein each of the first and second loading tubes is a double-layer tube.

8. The implant delivery device according to claim 2, wherein the loading unit further comprises a stent holder, wherein the stent holder is fixedly coupled to the loading unit and is used to engage with attachment lugs of the implant.

9. The implant delivery device according to claim 1, further comprising a third delivery catheter, wherein the third delivery catheter is movably inserted in the delivery assembly along the axial direction of the first flexible catheter.

10. An implant delivery system, comprising the implant delivery device according to any one of claims 1 to 9 and a driving device coupled to a proximal end of the delivery device, wherein the driving device is configured to drive the first and second flexible catheters to bend, respectively.
